Europäisches Patentamt

European Patent Office

Office européen des brevets

(19)

(11) Veröffentlichungsnummer: **0 359 981 B1**

(12) # EUROPÄISCHE PATENTSCHRIFT

(45) Veröffentlichungstag der Patentschrift: **28.07.93**

(51) Int. Cl.⁵: **A61K 45/06**, A61K 33/24, //(A61K33/24,31:685)

(21) Anmeldenummer: **89114818.1**

(22) Anmeldetag: **10.08.89**

(54) Pharmazeutische Kombinationspräparate und deren Verwendung als antineoplastische Arzneimittel.

(30) Priorität: **18.08.88 DE 3827974**

(43) Veröffentlichungstag der Anmeldung:
**28.03.90 Patentblatt 90/13**

(45) Bekanntmachung des Hinweises auf die Patenterteilung:
**28.07.93 Patentblatt 93/30**

(84) Benannte Vertragsstaaten:
**AT BE CH DE ES FR GB GR IT LI LU NL SE**

(56) Entgegenhaltungen:
**WO-A-84/01506**

**CHEMICAL ABSTRACTS, Band 94, 1981, Seite 62, Zusammenfassung Nr. 114601t, Columbus, Ohio, US; M. SLUYSER et al.: "Combined endocrine therapy and chemotherapy of mouse mammary tumors", & EUR. J. CANCER 1981, 17(2), 155-9**

(73) Patentinhaber: **BOEHRINGER MANNHEIM GMBH**
**Sandhofer Strasse 116**
**W-6800 Mannheim 31(DE)**

(72) Erfinder: **Grunicke, Hans Hermann, Prof.**
**Schützenstrasse 64**
**A-6060 Mils(AT)**
Erfinder: **Herrmann, Dieter, Dr.med.**
**An der Neckarspitze 13**
**W-6900 Heidelberg(DE)**
Erfinder: **Hofmann, Johann, Dr.**
**Giessenbach 328**
**A-6108 Scharnitz(AT)**
Erfinder: **Bosies, Elmar, Dr.phil.nat.**
**Delpstrasse 11**
**W-6904 Weinheim(DE)**

EP 0 359 981 B1

CHEMICAL ABSTRACTS, Band 100, 1984, Seite 33, Zusammenfassung Nr. 132230t, Columbus, Ohio, US; C. BENZ et al.: "tamoxifen and 5-fluorouracil in breast cancer: cytotoxic synergism in vitro", & CANCER RES. 1983, 43(11), 5298-303

CHEMICAL ABSTRACTS, Band 105, 1986, Seite 25, Zusammenfassung Nr. 202832v, Columbus, Ohio, US; V.V. FOMINA et al.: "New ways to improve the efficacy of tamoxifen in the treatment of mammary cancer", & IZV. AKAD. NAUK KAZ. SSR, SER. BIOL. 1986, (4) 67-70

CHEMICAL ABSTRACTS, Band 98, 1983, Seite 34, Zusammenfassung Nr. 119268g, Columbus, Ohio, US; L.D. MORRIS et al.: "Adjuvant treatment with adramycin, methotrexate and tamoxifen of DMBA induced tumors in the rat", & IRCS MED. SCI.: LIBR. COMPEND. 1982, 10(12), 1045

DIALOG 07028115, MEDLINE 89330115; J. HOFMANN: "Syneroistic annancement of the antioroliferative activity of cois-diamminedichloroplatinum(II) by the ether lipid analogue BM41440, an inhibitor if protein kinase C", & LIPIDS APRIL 1989, BAND 24, Nr. 4, SEITEN 312-317

CHEMICAL ABSTRACTS, Band 109, 1988, Seite 29, Zusammenfassung Nr. 204527f, Columbus, Ohio, US; J. HOFMANN et al.: "Enhancement of the antiproliferative effect of cis-diamminedichloroplatinum(II) and nitrogen mustard by inhibitors of protein kinase C", & INT. J. CANCER 1988, 42(3), 382-8

**Beschreibung**

Die vorliegende Erfindung betrifft pharmazeutische Kombinationspräparate und deren Anwendung als antineoplastische Arzneimittel.

Gegenstand der vorliegenden Erfindung ist ein pharmazeutisches Kombinationspräparat umfassend mindestens zwei Wirkstoffe, wobei der erste Wirkstoff ein Inhibitor der Proteinkinase-C ist, und der zweite Wirkstoff antineoplastische Wirkung aufweist, mit Ausnahme der Kombinationspräparate Tamoxifen/Cyclophosphamid, Tamoxifen/5-Fluoruracil und Tamoxifen/Adriamycin.

Die vorliegende Erfindung bezieht sich insbesondere auf Kombinationspräparate, die als Wirkstoffe Verbindungen enthalten, die als Inhibitoren der Proteinkinase C fungieren, in Kombination mit einem antineoplastisch aktiven Wirkstoff, wie beispielsweise mit Lipiden oder deren Derivate, mit cytostatisch wirksamen Verbindungen oder mit Verbindungen, die als Inhibitoren von Phospholipasen fungieren.

Insbesondere betrifft die Erfindung Kombinationspräparate von Proteinkinase-C-Inhibitoren mit cytostatisch wirksamen Verbindungen.

Einige Kombinationspräparate von Cytostatica sind aus dem Stand der Technik bereits bekannt. In Chem. Abstr., Band 94, 1981, 114601t und Chem. Abstr., Band 105, 1986, 202832v sind beispielsweise die Kombination von Tamoxifen und Cyclophosphamid beschrieben. Außerdem ist die Kombination von Tamoxifen und 5-Fluoruracil aus Chem. Abstr. Band 100, 1984, 132230t bekannt. Eine weitere Kombination von Tamoxifen und Adriamycin ist beschrieben in Chem. Abstr. Band 98, 1983, 119268g.

Die Verwendung von Chemotherapeutica ist in der antineoplastischen Therapie schon seit längerer Zeit ein anerkanntes und weit verbreitetes Behandlungsprinzip. Diese Chemotherapeutica werden eingesetzt, um maligne Zellen mit ungehemmten Wachstumsverhalten zu zerstören, wobei normale oder gesunde Zellen möglichst wenig geschädigt werden sollen. Als Chemotherapeutica kommen fast ausschließlich jedoch Cytostatica zum Einsatz, die im allgemeinen unspezifisch toxisch auf normale und maligne Zellen wirken und das Wachstum der Zellen hemmen. Diese Cytostatica haben eine sehr enge therapeutische Anwendungsbreite, was zu schwerwiegenden Nebenwirkungen führt.

Solche Nebenwirkungen sind beispielsweise Blutungen, Übelkeit, Erbrechen, Dysponoe, Allergien, Alopezien, Herzmuskelschädigungen, Herzrythmusstörungen, Perikarditis, periphäre und zentrale Neuropathien, Schmerzen, Nephropathien, Stomatitis, Diarrhoe, Fieber, Hautveränderungen, Infektionen, Herzinsuffizienzen, oder Veränderungen des Bewußtseinszustandes.

Die vorliegende Erfindung hat sich deshalb zur Aufgabe gestellt, die Wirkung von Chemotherapeutica zu steigern, ohne daß dies mit einer Steigerung der toxischen Effekte dieser Wirkstoffe einhergeht. Es sollen Arzneimittel zur Verfügung gestellt werden, die eine Verminderung der oben genannten Nebenwirkungen bei der Behandlung mit Chemotherapeutika bewirken. Ziel dabei ist es, die therapeutische Anwendungsbreite dieser Chemotherapeutica zu vergrößern. Im Falle der Cytostatica soll die antitumorale oder antiproliferative Wirkung verstärkt werden, so daß diese Cytostatica in geringeren Dosen verabreicht werden können und somit eine Verminderung oder Aufhebung der durch diese Mittel hervorgerufenen Nebenwirkungen erfolgt.

Es wurde nun überraschenderweise gefunden, daß Verbindungen, die Proteinkinasen C inhibieren, in Kombination mit antineoplastischen Wirkstoffen, wie z.B. mit Lipiden, Lipidanaloga, Cytostatica oder Inhibitoren von Phospolipasen eine synergistische Wirkung ausüben. Insbesondere wurde gefunden, daß bei der Kombination mit Cytostatika eine Verstärkung des antiproliferativen oder antitumoralen Effektes auftritt.

Im Sinne der vorliegenden Erfindung soll unter dem Begriff "Proteinkinase-C-Inhibitoren" solche Verbindungen verstanden werden, die die Calcium- und Phospholipid-abhängigen Proteinkinasen-C bzw. die entsprechenden Isoenzyme in zellfreien Extrakten oder in intakten Zellen hemmen (Nishizuka (1986), Science 233, 305 - 312); Nature (1988), 334, 662-665. Solche Stoffe können nach üblichen Verfahren aus Naturstoffen isoliert werden, oder auch synthetisch hergestellt werden. In diesem Sinne kommen beispielsweise die folgenden Verbindungen in Frage: Quercetin (3,3',4',5,7-Pentahydroxy-flavon, Horn, F. (1985), J. Biochem. 148, 533 - 538); Phorbolester, wie z. B. 12-O-Tetradeca-noylphorbol-13-acetat (TPA, Regazzi, R. (1986), Int. J. Cancer 37, 731 - 737); Tamoxifen (O'Brien et al. (1985), Cancer Research 45, 2462 - 2465); Staurosporin (Tamaoki, T. et al. (1986), Biochem. Biophys. Res. Comm. 135, 397 - 402); oder Lipidanaloga, wie beispielsweise schwefelhaltige Phospholipide, insbesondere Ilmofosin, oder Lysolecithine, insbesondere ET-18-OCH$_3$. Mit Hilfe der in Beispiel 1 beschriebenen allgemeinen Verfahren kann ohne weiteres experimentell festgestellt werden, ob eine Verbindung als Inhibitor der Proteinkinasen C wirkt und im Sinne der vorliegenden Erfindung verwendet werden kann.

Ilmofosin und dessen Verfahren zur Herstellung ist aus EP-B-0,050,327 bekannt. Die Verbindung ist dort als Beispiel 33 mit dem systematischen Namen 3-Hexadecylmercapto-2-methoxypropanol-1-phosphorsäuremonocholinester beschrieben. Ilmofosin gehört zur Gruppe der sog. Alkyllysolecithin-Derivate und ist als Verbindung mit cancerostatische Eigenschaften bekannt.

ET-18-OCH$_3$, dessen systematischer Name 4-Hydroxy-7-methoxy-N,N,N-trimethyl-3,5,9-trioxa-4-phosphaheptacosan-1-aminium-4-oxid lautet, ist aus DE PS 26 19 686 bekannt. Diese Verbindung ist dort als Antitumormittel beschrieben.

Als Proteinkinase-C-Inhibitoren im Sinne der vorliegenden Erfindung kommen insbesondere Ilmofosin und ET-18-OCH$_3$ in Frage. Weitere bevorzugte Verbindungen sind Quercetin, Tamoxifen und Staurosporin bzw. dessen chemisch modifizierten Derivate.

Zu der Gruppe der antineoplastisch aktiven Wirkstoffe gehören unter anderem Lipide oder Lipid-Analoga, wie z.B. Phospholipide, natürlich vorkommende oder auch synthetisch hergestellte Lipide, die einen Phosphatrest enthalten.

Hierzu zählen die Glycero- und die Sphingophosphatide sowie deren Derivate. Aus der Gruppe der synthetischen Derivate sind insbesondere folgende Strukturen zu nennen: 1-O-Alkylphospholipid-Derivate, wie zum Beispiel ET-18-OCH$_3$ (Weltzien, H.U. et al. in: Ether Lipids. Biochemical and Biomedical Aspects; Ed. Mangold, H.K. and Paltauf, F.; Academic Press, New York, 277-308);1-S-Alkylphospholipid-Derivate, wie zum Beispiel Ilmofosin (3-Hexadecyl-mercapto-2-methoxymethyl-propyl-1-phosphocholin); Alkyl-, Alkenyl- und Acylphosphocholin-bzw. ethanolamin-Derivate, wie beispielsweise Hexadecylphosphocholin (HPC); halogenierte Analoga von Alkyl-, Alkenyl- und Acylglycerol-Derivaten (Brackerts, H. (1987) Lipids 22, 897-903).

Zu den oben genannten Lipiden oder Lipid-Analoga gehören auch Lipide, die eine Kohlenhydratgruppe enthalten und als Glykolipide bezeichnet werden. Es werden Cerebroside und Ganglioside unterschieden. Ist der Galactoserest eines Cerebrosids mit Sulphat verestert, spricht man von Sulphatiden. Alle Sphingosin enthaltenden Lipide werden als Sphingolipide zusammengefaßt. Ferner kommen auch Neutrallipide, wie z.B. Triglyceride und Cholesteride sowie deren Derivate in Frage. Insbesondere sind in dieser Gruppe die O-Alkyl- und halogenierten Abkömmlinge des Triacylglycerols zu nennen.

Zu der Gruppe der antineoplastischen Wirkstoffe zählen ferner insbesondere die Chemotherapeutica. Darunter werden im folgenden körperfremde Substanzen verstanden, die geeignet sind und dazu verwendet werden, um Mikroorganismen, Parasiten (Antibiotika) oder Tumorzellen (Cytostatica) zu schädigen oder zu zerstören. Dabei sind insbesondere Cytostatica bzw. deren Derivate aus folgenden Cytostaticagruppen zu nennen: Alkylantien, wie z.B. Cyclophosphamid, Chlorambucil, Melphalan, Busulphan, N-Lost-Verbindungen, Mustargen; Metallkomplex-Cytostatica, wie z.B. Metallkomplexe des Platins, Palladiums oder Rutheniums, beispielsweise Cis-Diammino-dichloroplatin (II) (CDDP); Anti-metabolite, wie z.B. Methotrexat, 5-Fluoruracil, Cytorabin; Naturstoffe, wie beispielsweise Vinblastin, Vincristin, Vindesin usw.; Antibiotika, wie z.B. Dactinomycin, Daunorubicin, Doxorubizin, Bleomycin, Mitomycin usw.; Hormone und Hormonantagonisten, wie z.B. Diethylstilböstrol, Testolacton, Tamoxifen, Aminoglutethimid; sonstige Verbindungen, wie z.B. Hydroxyharnstoff oder Procarbacin, sowie Corticoide, wie z.B. Prednisolon.

Als antineoplastische Wirkstoffe im Sinne der vorliegenden Erfindung kommen insbesondere Platin-Komplex-Verbindungen, wie beispielsweise cis-Dichloro-diammin-Platin-(II) bzw. (IV), Mustargen und Doxorubicin (Adriamycin) in Frage.

Ferner gehören zu der Gruppe der antineoplastischen Wirkstoffe auch Verbindungen, die die Phospholipasen inhibieren, wie z.B. Mepacrin (Hofmann, S.L. et al. (1982) Arch. Biochem. Biophys. 215, 237-244), Antiphlogistica, wie z.B. Indomethacin etc., Neomycin, Psychopharmaka, Trifluoperazin etc..

In besonderen Fällen kann es auch vorkommen, daß eine Verbindung sowohl in die Gruppe der bereits bekannten antineoplastischen Wirkstoffe im Sinne der oben angegebenen Definition fällt, als auch der Gruppe der Proteinkinase-C-Inhibitoren zugeordnet werden kann. Dies ist beispielsweise der Fall für Ilmofosin und ET-18-OCH$_3$. Dies schließt jedoch die Kombinationsmöglichkeit mit anderen antineoplastischen Wirkstoffen nicht aus, solange mindestens einer der Wirkstoffe als Proteinkinase-C-Inhibitor fungiert.

Die Anwendung einer Kombinationstherapie mit Hilfe der pharmazeutischen Präparate der vorliegenden Erfindung bietet den Vorteil der synergistischen Verstärkung der antitumoralen Wirkung der Einzelsubstanzen. Dadurch ergibt sich die Möglichkeit der Reduktion der Dosen und damit der Toxizitäten der Einzelsubstanzen bei gleichzeitiger Erhaltung der antitumoralen Wirksamkeit bei Kombination der Einzelsubstanzen. Eine Kombinationstherapie der oben genannten Einzeltherapieprinzipien bietet ferner die Möglichkeit der Überwindung von Cytostatika-Resistenzen, wobei sowohl Substanzgruppenresistenzen als auch Mehrfachresistenzen (pleiotrope Cytostatikaresistenz) in Frage kommen.

Bei der Anwendung der Kombinationstherapie ist es möglich, die Wirkstoffe in einer sogenannten fixen Kombination, d.h. in einer einzigen pharmazeutischen Formulierung zu verabreichen, in der beide Wirkstoffe enthalten sind, oder eine sogenannte freie Kombination zu wählen, bei der die Wirkstoffe in Form von getrennten pharmazeutischen Formulierungen gleichzeitig oder aber auch nacheinander appliziert werden können. Die Herstellung solcher Kombinationspräparate erfolgt nach üblichen in der galenischen Tecknik bekannten Verfahren.

Sind die Wirkstoffe Feststoffe, so können die Wirkstoffe nach üblichen Verfahren zu festen Arzneimittelpräparaten (Tabletten, Pellets, Komprimetten, Geleekapseln) verarbeitet werden, indem man z.B. beide Wirkstoffe miteinander vermischt und mit üblichen Träger- oder Hilfsstoffen zusammen beispielsweise zu Tabletten verpreßt. Es ist aber auch möglich, die Wirkstoffe zusammen mit geeigneten pharmazeutischen Hilfsstoffen getrennt voneinander in einer verkaufsfertigen Verpackungseinheit zur Verfügung zu stellen, wobei die Verpackungseinheit die beiden Wirkstoffe in getrennten pharmazeutischen Formulierungen enthält.

Werden die Wirkstoffe in Form von Injektionslösungen zur Verfügung gestellt, so können diese die in Frage kommenden Wirkstoffkombinationen in lyophilisierter Form oder bereits in fertig injizierbarer gelöster Form enthalten. Prinzipiell ist es aber auch möglich, je eine parenterale Formulierung für jeden in Frage kommenden Wirkstoff in einer einzigen Verpackungseinheit zur Verfügung zu stellen, so daß die Injektionslösungen gegebenenfalls getrennt voneinander appliziert werden können. Bei Unverträglichkeiten der Wirkstoffe miteinander ist diese Form der Anwendung die bevorzugte Methode.

Im Falle der parenteralen Darreichungsform können die Wirkstoffe auch in Substanz, gegebenenfalls zusammen mit üblichen pharmazeutischen Hilfsstoffen, beispielsweise in lyophilisierter Form vorliegen und durch Zugabe von pharmazeutisch üblichen Injektionsmedien rekonstituiert oder solubilisiert werden.

Die pharmazeutischen Präparate kommen in flüssigen oder fester Form zur enteralen oder parenteralen Applikation. Hierbei kommen alle üblichen Applikationsformen in Frage, beispielsweise Tabletten, Kapseln, Dragees, Sirupe, Lösungen, Suspensionen. Als Injektionsmedium kommt vorzugsweise Wasser zur Anwendung, welches die bei Injektionslösungen üblichen Zusätze wie Stabilisierungsmittel, Lösungsvermittler und Puffer enthält. Derarige Zusätze sind z.B. Mannit, Tartrat- und Citratpuffer, Ethanol, Komplexbildner wie zum Beispiel Ethylendiamintetraessigsäure und deren nichttoxischen Salze, sowie hochmolekulare Polymere wie flüssiges Polyethylenoxid zur Viskositätsregelung. Flüssige Trägerstoffe für Injektionslösungen müssen steril sein und werden vorzugsweise in Ampullen abgefüllt. Feste Trägerstoffe sind zum Beispiel Stärke, Lactose, Kieselsäuren, höhermolekulare Fettsäuren wie Stearinsäure, Gelatine, Agar-Agar, Calciumphosphat, Magnesiumstereat, tierische und pflanzliche Fette, feste hochmolekulare Polymere wie Polyethylenglykole; für orale Applikation geeignete Zubereitungen können gewünschtenfalls Geschmacks-und Süßstoffe enthalten.

Die Dosierung kann von verschiedenen Faktoren, wie Applikationsweise, Spezies, Alter und individuellen Zustand abhängen. Die täglich zu verabreichenden Dosen liegen bei etwa 0.05 - 100 mg/kg Körpergewicht pro Einzelkomponente. Beispielsweise kann im Falle einer Kombination von CDDP und Quercetin eine Dosis von 1 - 10 mg/kg, insbesondere 3 mg/kg CDDP, oder 10 - 50 mg Quercetin, insbesondere 20 mg/kg verwendet werden. Die Menge des jeweiligen Wirkstoffes pro Darreichungsform variiert zwischen 5 und 1000 mg.

Bei den Kombinationspräparaten kann sich das Verhältnis zwischen den als Inhibitoren der Proteinkinase C fungierenden Wirkstoffen und den Lipiden, Lipidanaloga, Cytostatica oder Inhibitoren der Phospholipasen in einem weiten Bereich bewegen. So sind beispielsweise molare Verhältnisse zwischen 1:1000 und 1000:1 möglich, je nach Wirksamkeit der in Frage kommenden Wirkstoffe. Im Falle der Kombination mit Cytostatica ist ein Verhältnis zwischen 1:100 und 100:1 bevorzugt. Insbesondere kommt bei der Kombination von Ilmofosin oder ET-18-OCH3 mit cis-Diammino-dichloro-platin(II) ein Verhältnis von 1:50 bis 50:1, bevorzugt jedoch von 1:1 bis 50:1 in Frage.

Im Sinne der vorliegenden Erfindungen kommen beispielsweise die folgenden Kombinationspräparate in Frage:

| Proteinkinase-C-Inhibitor A | Antineoplastischer Wirkung B | Molares Verhältnis A : B |
|---|---|---|
| Quercetin | cis-DDP | ca. 100 : 1 |
| Quercetin | Mustargen | ca. 800 : 1 |
| Tamoxifen | cis-DDP | ca. 10 : 1 |
| Staurosporin | cis-DDP | ca. 1 : 100 |
| Ilmofosin | cis-DDP | ca. 10 : 1 |
| ET-18-OCH$_3$ | cis-DDP | ca. 10 : 1 |

Die folgenden Beispiele belegen die synergistische Wirkung einiger repräsentativer Kombinationspräparate, und stehen stellvertretend für den in den Ansprüchen näher gekennzeichneten Erfindungsgedanken:

5

Beispiel 1:

Allgemeines Verfahren zur Untersuchung von Verbindungen hinsichtlich ihrer Funktion als Inhibititor der Proteinkinase C'.

a) Reagentien

Pferdeserum und DMEM (Dulbeccos modified minimal essential medium) wurden von Boehringer Mannheim, FRG, bezogen. Cis-Diammino-dichloro-platin(II) stammte von Homburg Pharma, Frankfurt. [gamma-$^{32}$P]-ATP (10 Ci/mmol) und $^{32}$P-orthophosphat waren von Radiochemical Centre, Amersham, UK; GF/F-Filter und DE-52-Cellulose von Whatman, Clifton, NJ (USA); Leupeptin, Aprotinin, Quercetin (3,3',4',5,7-pentahydroxyflavon), Tamoxifen, 12-O-tetradecanoylphorbol-13-acetat (TPA), $\beta$-Glycerophosphat,Histon H1 (Typ III S), 3-N-Morpholino-propansulfonylsäure (MOPS), L-alpha-Phosphatidyl-L-serin und 1,2-sn-Diolein stammten von Sigma, München, FRG. Proteinkinase C wurde von Merck, Darmstadt, FRG bezogen. Tris(hydroxymethyl)-aminomethan (TRIS-HCl), Ethylenglykol-bis-(aminoethyl)-tetraessigsäure (EGTA), Natriumdodecylsulfat (SDS) und Triton X-100 waren von Serva, Heidelberg. Mustargen (HN2) wurde von Aldrich, Steinheim erhalten. Staurosporin stammte von Prof. Matter, Ciba-Geigy, Basel (CH).
Ilmofosin stammte von Boehringer Mannheim, FRG, und wurde wie von Bosies et al. (Lipids, (1987), 22, 947 - 951) beschrieben synthetisiert. Für die Untersuchungen wurde eine Stammlösung von 100 $\mu$g/ml Ilmofosin in DMEM plus 10% fötales Kälberserum (FCS) verwendet. Die Stammlösung wurde bei 4 °C gelagert.
Doxorubicin (Adriamycin, Adriablastin$^R$) stammte von Farmitalia/Carlo Erba GmbH, Freiburg, FRG.

b) Hemmung der Proteinkinase C (PK-C) - (in vitro Methode)

Proteinkinase C wurde aus Walker-Zellen durch Chromatographie an DEAE-Cellulose der Zellextrakte nach der von Kreutter et al. (J.Biol.Chem. 260, 5979-5984 (1985)) beschriebenen Methode angereichert. Zu den Extrakten wurde jedoch zusätzlich 1 mM Phenylmethan-sulfonylfluorid, 20 $\mu$g/ml Leupeptin und 2 $\mu$g/ml Aprotinin zugegeben. Die Proteinkinase C Aktivität wurde durch Messung des $^{32}$P-Einbaus von [gamma-$^{32}$P]-ATP in H1 Histon nach der Methode von Fabbro et al. (Arch. Biochem. Biophys. 239, 102 - 111 (1985)) bestimmt. Die Reaktionsmischung (125 $\mu$l) enthielt 0.5 $\mu$Ci [gamma-$^{32}$P]-ATP, 40 mM Tris-HCl, pH 7.4, 1mM CaCl$_2$, 700 $\mu$M EGTA, 50 $\mu$G Histon, 6.75 $\mu$g L-alpha-Phospatidyl-L-serin und 0.675 $\mu$g 1,2-s,n-diolein. Die Reaktionzeit betrug 10 Minuten bei 32 C. Die Enzymreaktion wurde durch Zugabe von 1 ml 20 % Trichloressigsäure (w/v) gestoppt. Das Protein wurde auf Whatman GF/F-Filterpapier ausgefällt und mit Hilfe eines Flüssigszintillationszählers gezählt.

c) Hemmung der Proteinkinase C' (PK-C') - (in vivo Methode) Phosphorylierung von ribosomalem Protein S6

Zellen wurden in DMEM mit 0.5 % Pferdeserum (v/v) über einen Zeitraum von 15 h gezüchtet und dann in phosphatfreiem Medium inkubiert. Nach 1 h wurden 4 $\mu$Ci/ml 32P-Orthophosphat und nach weiteren 30 Minuten 0.5 $\mu$M TPA (12-O-Tetradecanoylphorbol-13-acetat) und 50 $\mu$M Quercetin zugegeben. Die Zellen wurden in 50 mM Tris-HCl, pH 7.5, 25 mM KCl, 5 mM MgCl$_2$, 0.33 M Sacharose, 1 % Triton X-100 (v/v), 1 mM Phenylmethansulfonylchlorid, 20 $\mu$g/ml Leupeptin, 2 $\mu$g/ml Aprotinin und 80 mM $\beta$-Glycerophosphat lysiert. Das Lysat wurde nach 10 Minuten bei 0 C bei 30,000 g 10 Minuten lang zentrifugiert. Die Pellets wurden einmal durch Resuspension mit dem Lyse-Puffer gewaschen und anschließend bei 30,000 g zentrifugiert. Die Überstände wurden vereinigt und bei 100,000 g 3 Stunden lang zentrifugiert. Die Pellets wurden in 8 M Harnstoff resuspendiert und für 10 Minuten gekocht. Die Extrakte wurden durch eindimensionale SDS-Gelelektrophorese in 15-%-igen Polyacrylamid-Gelen analysiert (Laemmli, U.K. (1970), Nature 227, 680-685). Nach Blotten des Gels auf Nitrocellulose wurde das S6-Protein identifiziert durch Zugabe von anti-S6-Antiserum.

Beispiel 2:

Quercetin als Inhibitor der Proteinkinase C

Wie in Beispiel 1 näher beschrieben wurde der Einfluß von Quercetin auf die Proteinkinase C und auf die Vermehrung von Walker-Carzinoma-Zellen in Kultur untersucht. Quercetin wurde in Dimehtylsulfoxid (DMSO) solubilisiert. Die Lösung in DMSO wurde zur Ansatzmischung bis zu einer DMSO-Endkonzentration von 1 % zugegeben. Eine äquivalente Menge von reinem DMSO wurde zu der Kontrollgruppen zugegeben. Der Einfluß von Quercetin auf die Zellvermehrung wurde durch Zugabe des Wirkstoffes in DMSO zum Kulturmedium bis zu einer Endkonzentration von 0.1 % untersucht. Die Zellen wurden in Gegenwart des Wirkstoffes 48 Stunden lang gezüchtet. Die Kontrollgruppe erhielt lediglich reines DMSO. 100 % der Proteinkinase C Aktivität entspricht 44.8 pmol/min auf H1 übertragenem $^{32}$P.

Aus den erhaltenen Daten geht hervor, daß Quercetin als Inhibitor der Proteinkinase C fungiert ($IC_{50}$ = 25 $\mu$M).

Beispiel 3:

Tamoxifen als Inhibitor der Proteinkinase C

Analog wie in Beispiel 2 beschrieben wurde der Einfluß von Tamoxifen auf die Proteinkinase C untersucht. Aus den erhaltenen Daten konnte der $IC_{50}$-Wert zu 11.20 $\mu$M bestimmt werden.

Beispiel 4:

Staurosporin als Inhibitor der Proteinkinase

Analog wie in Beispiel 2 beschrieben wurde der Einfluß von Staurosporin auf die Proteinkinase C untersucht. Der $IC_{50}$-Wert konnte zu 0.048 $\mu$M bestimmt werden.

Beispiel 5:

Ilmofosin als Inhibitor der Proteinkinase C

Analog wie in Beispiel 2 beschrieben wurde der Effekt von Ilmofosin auf die Proteinkinase C untersucht. Der $IC_{50}$-Wert beträgt 20 $\mu$M.

Beispiel 6:

ET-18-OCH$_3$ als Inhibitor der Proteinkinase C

Analog wie in Beispiel 2 beschrieben wurde der Einfluß von ET-18-OCH$_3$ auf die Proteinkinase C untersucht. ET-18-OCH3 inhibiert ebenso wie Ilmofosin die Proteinkinase C ($IC_{50}$ = 24.8 $\mu$M).

Beispiel 7:

Allgemeines Verfahren zur Bestimmung des synergistischen Effektes einer Kombination aus Proteinkinase-C-Inhibitor und einem antineoplastischen Wirkstoff

a) Walker Karzinoma-Zellen von Ratten wurden in Suspensionen von DMEM (Dulbeccos Modified Minimal Essential Medium) mit einem 10 %-igen Anteil (v/v) von Pferdeserum und 25 mM MOPS-Puffer (pH 7.35 bei 20 C) bei einer Temperatur von 36.8 C gezüchtet. Dosis-Wirkungs-Kurven für einzelne Wirkstoffe oder Wirkstoffkombinationen wurden erhalten durch Zugabe der entsprechenden Wirkstoffe zu einer Suspenion von Walker-Zellen ($10^5$ Zellen/ml). Nach einer Inkubationszeit von 48 h wurden die Zellen mit Hilfe eines elektronischen Zählers (Coulter-Electronics, Luton, UK) gezählt. Die Vermehrung der Zellen (M) wurde nach folgender Formel berechnet: M = $(T_t-T_0)/(C_t-C_0)$*100, wobei C für die unbehandelten Kontroll-Zellen steht, T die Anzahl der behandelten Zellen bedeutet, und die Indices 0 und t für die Zahl der Zellen zum Zeitpunkt 0 und nach 48 h steht.

Nach der von Chou und Talalay (Eur. J. Biochem. (1981), 115, 207 - 216 und Advances Enzyme Regul. (1984), 22, 27 - 54) beschriebenen Methode wurde der synergistische Effekt der Wirkstoffkombinationen bestimmt. Die zur Berechnung verwendeten Daten beruhen auf mindestens drei verschiedenen Experimenten. Das Berechnungsprogramm wurde bei Elsevier Biosoftware, Cambridge, UK bezogen (Dose effect analysis with microcomputers).

b) [$^3$H]-Thymidin Einbau

Der cytostatische bzw. cytotoxische Effekt der Wirkstoffe bzw. Wirkstoffkombinationen auf MethA-Fibrosarkoma-Zellen wurde in vitro anhand des verringerten [$^3$H]-Thymidineinbaus untersucht. Die Zellen wurden bis zu einer Endkonzentration von $5 \times 10^4$ /ml in Abwesenheit der Wirkstoffe suspendiert in DMEM, 10 % FCS, 50 $\mu$M 2-Mercaptoethanol, 100 U/ml Penicillin, 100 $\mu$g/ml Streptomycin. Die Wirkstoffe wurden in einem Volumen von 20 $\mu$l zu den Zellen hinzugefügt. Pro Konzentration wurden 6 Kulturen zu 0.2 ml angesetzt und in Mikrotiterplatten bei feuchtem Klima inkubiert. Die Kulturen wurden 3 Std. mit 1 $\mu$Ci (27 kBq/Zelle) [Methyl-$^3$H]-Thymidin (spezifische Aktivität 5 Ci/mmol) gepulst. Die Proben wurden anschließend geerntet und mehrfach gewaschen. Die Filterplatten wurden getrocknet und in Szintillationsgläschen überführt. Der radioaktive Einbau wurde nach Zugabe von Rotiszint gemessen.

Beispiel 8:

Synergistischer Effekt von Quercetin und cis-Diamminodichloro-platin (II) (cis-DDP)

Wie in Beispiel 7a beschrieben wurde der Einfluß von cis-DDP, Quercetin und einer Mischung von Quercetin/cis-DDP (molares Verhältnis 100:1) auf Walker-Sarkoma-Zellen von Ratten untersucht. Die Zellen wurden in Gegenwart der jeweiligen Wirkstoffen für eine Dauer von 48 Stunden gezüchtet. Das Ergebnis geht aus Tab. 1 hervor.

Beispiel 9:

Synergistische Kombination von Quercetin und Mustargen

Wie in Beispiel 7a angegeben wurde der Einfluß von Quercetin, Mustargen und einer Mischung von Quercetin/Mustargen (molares Verhältnis 800:1) auf Walker-Sarkoma-Zellen von Ratten untersucht. Das Ergebnis geht aus Tab. 1 hervor.

Beispiel 10:

Synergistische Kombination von Tamoxifen und cis-DDP

Wie in Beispiel 7a angegeben wurde der Einfluß von Tamoxifen, cis-DDP und einer Mischung aus Tamoxifen/cis-DDP (molares Verhältnis 10:1) auf Walker-Sarkoma-Zellen von Ratten untersucht. Das Ergebnis geht aus Tab. 1 hervor.

Beispiel 11:

Synergistische Kombination von Staurosporin und cis-DDP

Wie in Beispiel 7a angegeben wurde der Einfluß von Staurosporin, cis-DDP und einer Mischung aus Staurosporin/cis-DDP (molares Verhältnis 1:100) auf Walker-Sarkoma-Zellen von Ratten untersucht. Das Ergebnis ist in Tab. 1 angegeben.

Beispiel 12:

Synergistische Kombination von Ilmofosin und cis-DDP

Wie in Beispiel 7a angegeben wurde der Einfluß von Ilmofosin, cis-DDP und einer Mischung von Ilmofosin/cis-DDP (molares Verhältnis 10:1) auf Walker-Sarkoma-Zellen von Ratten untersucht. Das Ergebnis geht aus Tab. 1 hervor.

Beispiel 13:

Synergistische Kombination von ET-18-OCH$_3$ und cis-DDP

Wie in Beispiel 7a angegeben wurde der Einfluß von ET-18-OCH$_3$, cis-DDP und einer Mischung von ET-18-OCH$_3$ (molares Verhältnis 10:1) auf Walker-Sarkoma-Zellen von Ratten untersucht. Ähnlich wie in Beispiel 12 beschrieben ist auch in diesem Falle eine synergistische Wirkung festzustellen (vgl. Tab. 1).

Tab. 1  Hemmung der zellulären Replikation und Verstärkung des antiproliferativen Effektes; Zusammenstellung der $IC_{50}$-Werte:

| Bsp. | Protein-kinase C Inhibitor | Hemmung der Proteinkinase C $IC_{50}$ [$\mu$M] | (1) Zell-Proliferation $IC_{50}$ [$\mu$M] | $IC_{50}$ [$\mu$M] (1) Zell-Proliferation in Kombination mit cis-DDP | (2) Art der Aktivität |
|------|------|------|------|------|------|
| 2, 8 | Quercetin | 25 | 23 | 3.8 | Synergismus |
| 2, 9 | Quercetin | 25 | 23 | | Synergismus |
| 3, 10 | Tamoxifen | 11.20 | 12.44 | 2.24 | Synergismus |
| 4, 11 | Staurosporin | 0.048 | 0.04 | 0.004 | Synergismus |
| 5, 12 | Ilmofosin | 0.56 | 20 | 2 | Synergismus |
| 6, 13 | Et-18-OCH₃ | 24.8 | 5.8 | 1.7 | Synergismus |
| | cis-DDP | > 1000 | 0.23 | – | – |

(1)  Unter dem $IC_{50}$-Wert versteht man diejenige
Konzentra-tion des Inhibitors, bei der eine 50%-ige
Hemmung der Proteinkinase C oder der Zellproliferation erreicht wird.

(2)  Die Berechnungsgrundlage hinsichtlich des synergistischen Effektes erfolgte nach der Methode von Chou
und Talalay (Advances Enzyme Regul. 22, 27 - 54
(1984)).

Beispiel 14:

Wie in dem allgemeinen Verfahren in Beispiel 7b) beschrieben wurden die $IC_{50}$-Werte für eine
Kombination von Ilmofosin mit CDDP bzw. Doxorubicin bestimmt. Das Ergebnis geht aus der Tabelle 2
hervor.

Tab. 2

| Hemmung der Tumorzellen-Proliferation | | | |
|------|------|------|------|
| Inhibitor PK-C A | Antineopl. Wirkstoff B | Verhältnis A:B | $IC_{50}$ [$\mu$g/ml] |
| Ilmofosin | CDDP | 100 : 1 | 1.09 |
| Ilmofosin | Doxorubicin | 100 : 1 | 1.11 |
| Ilmofosin | - | - | 1.06 |

Beispiel 15:

Herstellung von pharmazeutischen Formulierungen

Die als Wirkstoffe ausgewählten Verbindungen A und B können in verschiedenen galenischen Formulierungen verwendet werden. Die nachstehend angegebenen Beispiele betreffen galenische Zubereitungen, die einen mit dem Buchstaben A bezeichneten Wirkstoff als Proteinkinase-C-Inhibitor enthält und einen mit dem Buchstaben B bezeichneten antineoplastischen Wirkstoff.

a) Tabletten

| Mischung I | | Mischung II | |
|---|---|---|---|
| Wirkstoff A | 50 mg | Wirkstoff B | 50 mg |
| Stärke | 180 mg | Silicieundioxid | 100 mg |
| Magnesiumstearat | 20 mg | Laktose | 100 mg |
| | | Aerosil | 5 mg |

Mischung I und II werden getrennt voneinander trocken oder feucht granuliert. Anschließend werden sie unter Zusatz von 5 mg Talkum miteinander vermischt und zu Tabletten verpreßt.

b) Kapseln

| Mischung I | | Mischung II | |
|---|---|---|---|
| Wirkstoff A | 50 mg | Wirkstoff B | 200 mg |
| Lactose | 110 mg | Polyvinylpyrolidon | 10 mg |
| Maisstärke | 20 mg | Maisstärke | 100 mg |
| Gelantine | 8 mg | Ceetina HR | 10 mg |
| Magnesiumstearat | 12 mg | | |

Die beiden Mischungen I und II werden getrennt voneinander nach üblichen Verfahren granuliert. In einem geeigneten Mixer werden die beiden Granulate in dem gemischten Mischungsverhältnis miteinander vermischt und das Pulver im Mixer mit Talkum versetzt. Anschließend wird die Mischung maschinell in Hartgelantinekapseln abgefüllt.

c) Injektionslösung i.m. oder i.v.

Eine spritzfertige i.v.-Injektionslösung enthält

| | |
|---|---|
| Wirkstoff A | 50 mg |
| Wirkstoff B | 100 mg |
| Natriumchlorid | 20 mg |
| Natriumacelat | 6 mg |
| dest. Wasser ad | 5 ml |

Eine spritzfertige i.m.-Injektionslösung enthält

| | |
|---|---|
| Wirkstoff A | 100 mg |
| Wirkstoff B | 100 mg |
| Benzylbenzoat | 1 g |
| Injektionsöl | 5 ml |

EP 0 359 981 B1

**Patentansprüche**

1. Pharmazeutisches Kombinationspräparat umfassend mindestens zwei Wirkstoffe, dadurch gekennzeichnet, daß der erste Wirkstoff ein Inhibitor der Proteinkinase-C ist, und der zweite Wirkstoff antineoplastische Wirkung aufweist, sowie pharmakologisch verträgliche Träger- oder Hilfsstoffe, mit Ausnahme der Kombinationspräparate Tamoxifen/Cyclophosphamid, Tamoxifen/5-Fluoruracil und Tamoxifen/Adriamycin.

2. Pharmazeutisches Kombinationspräparat gemäß Anspruch 1, dadurch gekennzeichnet, daß der Wirkstoff, der als Inhibitor der Proteinkinase C fungiert, eine Lipidanaloge Verbindung ist.

3. Pharmazeutisches Kombinationspräparat gemäß Anspruch 1, dadurch gekennzeichnet, daß der Wirkstoff, der als Inhibitor der Proteinkinase C fungiert, Quercetin, Tamoxifen, Ilmofosin, ET-18-OCH$_3$ oder Staurosporin ist.

4. Pharmazeutisches Kombinationspräparat gemäß Anspruch 1, dadurch gekennzeichnet, daß der zweite Wirkstoff ein Lipid oder ein Lipid-analoge Verbindung ist.

5. Pharmazeutisches Kombinationspräparat gemäß Anspruch 4, dadurch gekennzeichnet, daß der Wirkstoff Hexadecylphosphocholin oder ein halogeniertes Alkyl-, Alkenyl- oder Acylglycerol-Derivat ist.

6. Pharmazeutisches Kombinationspräparat gemäß Anspruch 1, dadurch gekennzeichnet, daß der zweite Wirkstoff ein Cytostatikum ist.

7. Pharmazeutisches Kombinationspräparat gemäß Anspruch 6, dadurch gekennzeichnet, daß die Cytostatica Metallkomplex-Verbindungen, insbesondere des Platins, Hormonantagonisten oder Corticoide sind.

8. Pharmazeutisches Kombinationspräparat gemäß Anspruch 6, dadurch gekennzeichnet, daß die Cytostatica Chlorambucil, cis-Diammino-dichloro-platin(II), Methotrexat, oder Bleomycin sind.

9. Pharmazeutisches Kombinationspräparat gemäß einem der Ansprüche 1-4 oder 6, dadurch gekennzeichnet, daß es Ilmofosin oder cis-Diammino-dichloro-platin(II) enthält.

10. Verfahren zur Herstellung von pharmazeutischen Kombinationspräparaten nach den Ansprüchen 1-9, dadurch gekennzeichnet, daß man den jeweiligen Wirkstoff zusammen mit pharmazeutisch üblichen Träger- oder Hilfsstoffen vermischt und die betreffenden Präparate in Form von Kombinationspräparaten zur Verfügung stellt.

11. Verwendung von Verbindungen mit Proteinkinase-C inhibierender Wirkung oder von antineoplastischen Wirkstoffen zur Herstellung von pharmazeutischen Kombinationspräparaten wie in den Ansprüchen 1 - 9 näher gekennzeichnet.

12. Verfahren zur Herstellung eines pharmazeutischen Kombinationspräparates mit synergistischer antineoplastischer Wirkung mit Ausnahme der Kombinationspräparate Tamoxifen/Cyclophosphamid, Tamoxifen/5-Fluoruracil und Tamoxifen/Adriamycin., dadurch gekennzeichnet, daß man den Wirkstoff mit antineoplastischer Wirkung zusammen mit einem Proteinkinase-C-inhibierenden Wirkstoff konfektioniert.

13. Verfahren zur Herstellung eines einen ersten und mindestens einen zweiten Wirkstoff enthaltenden Kombinationspräparates mit synergistischer antineoplastischer Wirkung, umfassend die folgenden Verfahrensschritte:
    a) Bestimmung der Proteinkinase-C-inhibierenden Wirkung des ersten Wirkstoffes,
    b) Bestimmung des synergistischen Effektes dieses ersten Wirkstoffes in Kombination mit einem zweiten eine antineoplastische Wirkung aufweisenden Wirkstoffes, und
    c) Konfektionierung dieses ersten und zweiten Wirkstoffes in Form eines pharmazeutischen Kombinationspräparates.

11

**Claims**

1. Pharmaceutical combination preparation comprising at least two active materials, characterised in that the first active material is an inhibitor of protein kinase C and the second active material has an anti-neoplastic action, as well as pharmacologically compatible carrier or adjuvant materials, with the exception of the combination preparations tamoxifen/cyclophosphamide, tamoxifen/5-fluoruracil and tamoxifen/adriamycin.

2. Pharmaceutical combination preparation according to claim 1, characterised in that the active material which functions as inhibitor of protein kinase C is a lipid-analogous compound.

3. Pharmaceutical combination preparation according to claim 1, characterised in that the active material which functions as inhibitor of protein kinase C is querctin, tamoxifen, ilmofosin, ET-18-OCH$_3$ or staurosporin.

4. Pharmaceutical combination preparation according to claim 1, characterised in that the second active material is a lipid or lipid-analogous compound.

5. Pharmaceutical combination preparation according to claim 4, characterised in that the active material is hexadecylphosphocholine or a halogenated alkyl-, alkenyl- or acyl-glycerol derivative.

6. Pharmaceutical combination preparation according to claim 1, characterised in that the second active material is a cytostatic.

7. Pharmaceutical combination preparation according to claim 6, characterised in that the cytostatics are metal complex compounds, especially of platinum, hormone antagonists or corticoids.

8. Pharmaceutical combination preparation according to claim 6, characterised in that the cytostatics are chlorambucil, cis-diamminedichloroplatinum(II), methotrexate or bleomycin.

9. Pharmaceutical combination preparation according to one of claims 1 - 4 or 6, characterised in that it contains ilmofosin or cis-diamminedichloroplatinum(II).

10. Process for the preparation of pharmaceutical combination preparations according to claims 1 - 9, characterised in that one mixes the active material in question together with pharmaceutically usual carrier or adjuvant materials and makes the preparation in question available in the form of combination preparations.

11. Use of compounds with protein kinase C inhibiting action or of anti-neoplastic active materials for the preparation of pharmaceutical combination preparations as characterised in more detail in claims 1 - 9.

12. Process for the preparation of a pharmaceutical combination preparation with synergistic anti-neoplastic action with the exception of the combination preparations tamoxifen/cyclophosphamide, tamoxifen/5-fluoruracil and tamoxifen/adriamycin, characterised in that one makes up the active material with anti-neoplastic action together with a protein kinase C-inhibiting active material.

13. Process for the preparation of a combination preparation with synergistic anti-neoplastic action containing a first and at least a second active material, comprising the following process steps:
    a) determination of the protein kinase C-inhibiting action of the first active material,
    b) determination of the synergistic effect of this first active material in combination with a second active material displaying an anti-neoplastic action and
    c) making up of this first and second active material in the form of a pharmaceutical combination preparation.

**Revendications**

1. Préparation pharmaceutique combinée, comprenant au moins deux principes actifs, caractérisée en ce que le premier principe actif est un inhibiteur de la protéine-kinase C, et que le second principe actif présente une activité antinéoplasique, cette préparation comprenant les supports ou adjuvants pharmacologiquement acceptables, à l'exclusion des préparations combinées tamoxifène/cyclophosphamide. tamoxifène/5-fluorouracile et tamoxifène/adriamycine.

**2.** Préparation pharmaceutique combinée selon la revendication 1, caractérisée en ce que le principe actif, qui agit comme inhibiteur de la protéine-kinase C, est un composé analogue lipidique.

**3.** Préparation pharmaceutique combinée selon la revendication 1, caractérisée en ce que le principe actif, qui agit comme inhibiteur de la protéine-kinase C, est la quercetine, le tamoxifène, l'ilmofosine, l'ET-18-OCH$_3$ ou la staurosporine.

**4.** Préparation pharmaceutique combinée selon la revendication 1, caractérisée en ce que le second principe actif est un lipide ou un analogue lipidique.

**5.** Préparation pharmaceutique combinée selon la revendication 4, caractérisée en ce que le principe actif est de l'hexadécylphosphocholine ou un dérivé halogéné d'alkyl-, alcényl-ou acylglycérol.

**6.** Préparation pharmaceutique combinée selon la revendication 1, caractérisée en ce que le second principe actif est un agent cytostatique.

**7.** Préparation pharmaceutique combinée selon la revendication 6, caractérisée en ce que l'agent cytostatique est un composé complexe métallique, en particulier un composé complexe métallique du platine, un antagoniste hormonal ou un corticoïde.

**8.** Préparation pharmaceutique combinée selon la revendication 6, caractérisée en ce que l'agent cytostatique est le chlorambucile, le cis-diamino-dichloroplatine (II), le méthotrexate ou la bléomycine.

**9.** Préparation pharmaceutique combinée selon l'une quelconque des revendications 1-4 ou 6, caractérisée en ce qu'elle contient de l'ilmofosine ou du cis-diamino-dichloroplatine (II).

**10.** Procédé d'obtention de préparations pharmaceutiques combinées selon les revendications 1-9, caractérisé en ce que l'on mélange le principe actif considéré avec des supports ou adjuvants galéniques usuels, et l'on fournit les préparations concernées sous la forme de préparations combinées.

**11.** Utilisation de composés ayant une activité inhibitrice de la protéine-kinase C ou de principes actifs antinéoplasiques, pour obtenir des préparations pharmaceutiques combinées telles que caractérisées en détail aux revendications 1-9.

**12.** Procédé d'obtention d'une préparation pharmaceutique combinée ayant une activité synergique antinéoplasique, à l'exclusion des préparations combinées tamoxifène/cyclophosphamide, tamoxifène/5-fluorouracile et tamoxifène/adriamycine, caractérisé en ce que l'on produit le principe actif ayant une activité néoplasique conjointement avec un principe actif ayant une activité inhibitrice de la protéine-kinase C.

**13.** Procédé d'obtention d'une préparation combinée contenant un premier principe actif et au moins un second principe actif, comprenant les étapes suivantes :
a) détermination de l'activité inhibitrice de la protéine-kinase C du premier principe actif,
b) détermination de l'action synergique de ce premier principe actif en combinaison avec un second principe actif ayant une activité néoplasique, et
c) obtention de ces premier et second principes actifs sous la forme d'une préparation pharmaceuti-que combinée.